# EUROPÄISCHE PATENTSCHRIFT

(11) **EP 3 130 325 B1**
(45) Veröffentlichungstag und Bekanntmachung des Hinweises auf die Patenterteilung: **14.11.2018**
(21) Anmeldenummer: 15180817.7
(22) Anmeldetag: 12.08.2015
(51) Int. Cl.: A61K 6/00

(54) **MITTEL FÜR DIE DENTIN- UND SCHMELZKONDITIONIERUNG**
AGENT FOR DENTIN AND ENAMEL CONDITIONING
MOYEN POUR LE CONDITIONNEMENT DE L'EMAIL ET DE LA DENTINE

(43) Veröffentlichungstag der Anmeldung: 15.02.2017
(73) Patentinhaber: Ivoclar Vivadent AG, 9494 Schaan (LI)
(72) Erfinder: CATEL, Delphine, 9475 Rans (CH); BOCK, Thorsten, 6800 Feldkirch (AT)
(74) Vertreter: Uexküll & Stolberg

(56) Entgegenhaltungen:
- EP-A1- 2 626 057
- US-A- 4 964 911
- US-A- 5 270 351
- US-A- 5 354 827

## Beschreibung

Die Erfindung betrifft Formulierungen zur Konditionierung der Zahnhartsubstanz (Dentin & Schmelz), um einen dauerhaften und starken Verbund mit dentalen Restaurationsmaterialien zu erzielen. Die Formulierungen zeichnen sich durch einfache Handhabung, hohe Fehlertoleranz und verbesserte Langzeitbeständigkeit des Verbundes aus.

Bei der mechanischen Bearbeitung ("Bohren") von Zähnen bildet sich auf der Zahnoberfläche eine sogenannte Schmierschicht, die primär aus mikroskopischen Bohrrückständen besteht. Die Schmierschicht behindert die Haftung der Werkstoffe, die zur anschließenden Restauration des Zahns eingesetzt werden. Das Standardverfahren zur Entfernung der Schmierschicht ist die Behandlung der Zahnoberfläche mit 35-37%iger Phosphorsäure. Dieses Verfahren wird auch als "Total-Etch" (TE) oder "Etch&Rinse" Verfahren bezeichnet. Das Entfernen der Schmierschicht ermöglicht dem im Anschluss aufgetragenen Dentaladhäsiv den Kontakt mit der intakten und mechanisch belastbaren Zahnoberfläche. Auf Zahnschmelz erzeugt Phosphorsäure ein ausgeprägtes mikroretentives Ätzmuster und gestattet so den mikromechanischen Haftverbund mit dem Adhäsivsystem. Auf Dentin werden durch das Konditionierungsmittel die Dentintubuli von der Schmierschicht befreit und Kollagenfasern freigelegt. Die Infiltration der Tubuli und der Kollagenfasern durch das Adhäsiv führt zu einer mikromechanischen Bindung (Pashley, D.H., The evolution of dentin bonding from no-etch to total-etch to self-etch, Adhes. Tech. Sol. 2002, 1, 1-5).

Der übliche Behandlungsvorgang der direkten restaurativen Therapie mit einem TE-Adhäsiv sieht folgende Arbeitsschritte vor: (1) Mechanisches Entfernen kariösen Gewebes; (2) Ätzen der Zahnoberfläche mit Phosphorsäure; (3) Abspülen der Phosphorsäure mit Wasser; (4) Entfernen überschüssigen Wassers von der Kavitätenoberfläche mit einem dentalen Luftbläser ("trocknen"); (5) Applikation des Adhäsivs; (6) Verblasen des Lösemittels; (7) Lichthärten des Adhäsivs; (8) Applikation des Füllungskomposits und ggf. weitere Schritte zur Fertigstellung der Restauration.

Ein kritischer Schritt dieses Verfahrens ist die Trocknung der Zahnoberfläche nach dem Abspülen der Phosphorsäure. Es hat sich gezeigt, dass unabhängig vom eingesetzten Adhäsiv die Haltbarkeit der Restauration maßgeblich von der Feuchte der Dentinoberfläche abhängt. Um eine hohe Haftung zu erzielen muss vor der Applikation des Adhäsivs eine bestimmte Restfeuchte eingestellt werden ("Wet-Bonding-Technik"). Jedoch legt Phosphorsäure durch ihre stark demineralisierende Wirkung die im Dentin vorhandenen Kollagenfibrillen frei. Auf solchermaßen präpariertem Dentin führt schon geringfügig zu langes Trocknen mit dem Luftbläser zum Kollabieren der freigelegten Kollagenfibrillen, wodurch der direkte Kontakt mit intakter Zahnhartsubstanz, die Infiltration der Kollagenfasern und der durch das Ätzen erweiterten Tubuli mit Adhäsiv erschwert wird. Dies hat eine starke Herabsetzung des initialen Haftverbundes, mangelnde Langzeitbeständigkeit des Dentinverbundes (insbesondere an mechanisch stark belasteten Stellen wie, z.B. bei Zahnhöckeraufbauten) und eine erhöhte Mobilität des Dentinliquors unter externem Stimulus und damit eine erhöhte Inzidenz post-operativer Empfindlichkeiten zur Folge (Pashley, D.H., The evolution of dentin bonding from no-etch to total-etch to self-etch, Adhes. Tech. Sol. 2002, 1, 1-5). Diese Beschwerden sind für den Patienten sehr unangenehm und für den Zahnarzt aufwändig, weil die Patienten nachbehandelt werden müssen.

Wie das Übertrocknen hat jedoch auch das unzureichende Trocknen des Dentins negative Folgen. Wird das Adhäsiv auf zu feuchtes Dentin aufgetragen, kommt es zur Phasenseparation in besser und weniger gut wasserverträgliche Adhäsivkomponenten. Da die üblichen Lichtinitiatorsysteme nur schlecht wasserverträglich sind, ist die Polymerisationsfähigkeit des Adhäsivs in wasserreichen Phasen eingeschränkt. Die resultierende Adhäsivschicht ist bezüglich Zusammensetzung und Polymerisationsgrad heterogen, was einen reduzierten Haftverbund und einen unzureichendem Verschluss der Tubuli zur Folge hat.

Die Problematik wird dadurch zusätzlich verkompliziert, dass die optimale Feuchte zur Erzielung einer idealen Dentinhaftung stark vom verwendeten Adhäsiv abhängt. Trotz der großen Bedeutung des Total-Etch-Protokolls für die restaurative Zahnheilkunde wurde daher intensiv nach Alternativen gesucht.

Ein anderes Problem des Total-Etch-Protokolls sind die genau einzuhaltenden, aber unterschiedlichen Einwirkdauern von Phosphorsäure auf Dentin (15s) und Schmelz (30s). Da der säurebeständigere Schmelz eine längere Kontaktdauer mit Phosphorsäure zur Konditionierung benötigt, wird dieser üblicherweise zuerst für 15s mit Phosphorsäure behandelt, und danach wird die Säure auch auf das Dentin aufgetragen und die Behandlung für weitere 15s fortgesetzt (Brady, L.A., Total-etch or self-etch: the debate continues, DentistryIQ, 2011**).**

Bei der Applikation des Ätzmittels auf den Schmelz kann aber vor allem bei kleineren Kavitäten ein Dentinkontakt nicht vollständig ausgeschlossen werden, was ein zu langes Einwirken der Säure auf das Dentin zur Folge hat. Zu langes Ätzen von Dentin bewirkt eine übermäßige Aufweitung der Dentintubuli und eine starke Demineralisierung der Dentinoberfläche ("Überätzen"). Ein vollständiger Verschluss stark aufgeweiteter Dentintubuli mit Adhäsiv ist jedoch bestenfalls schwierig, wie die deutlich erhöhte Inzidenz postoperativer Empfindlichkeiten nach Überätzen nahelegt (Brady, L.A., Total-etch or self-etch: the debate continues, DentistryIQ, 2011**).**

Weiterhin ist bekannt, dass Phosphorsäure die in kollagenösem Gewebe natürlich vorkommenden Matrix-Metalloproteasen (MMP) und Cystein-Cathepsine aktiviert. Nach der adhäsiven Versorgung bedingen diese Enzyme, auch bei sonst korrekt durchgeführter adhäsiver Versorgung der Kavität, den sukzessiven Abbau des freigelegten Kollagennetzwerkes. Da das Kollagennetzwerk in der Adhäsivschicht ein merkliches Volumen einnimmt, hinterlässt sein Abbau Hohlräume und mindert die mechanische Belastbarkeit des Zahn-Adhäsiv-Verbundes. Zahlreiche *in vitro* Untersuchungen zum enzymatischen Abbau dentinalen Kollagens in restauriertem Zahngewebe haben eine deutliche Reduktion der Verbundwirkung schon nach verhältnismäßig kurzer Zeit gezeigt (Tjaderhane, L. et al., Optimizing dentin bond durability: Control of collagen degradation by matrix metalloproteinases and cysteine cathepsins, Dent. Mater. 2013, 29, 116-135).

Wegen der genannten Probleme ist das Total-Etch-Protokoll empfindlich für Anwendungsfehler und ergibt häufig nicht das gewünschte Ergebnis. In der Hoffnung, die Nachteile der Phosphorsäureanwendung zu vermeiden, wurden zahlreiche alternative Konditionierungsmittel entwickelt. Johnson, G. et al., Dentin bonding systems: a review of current products and techniques, The Journal of the American Dental Association, 1991, 122, 34-41, untersuchen 11 verschiedene, kommerziell erhältliche Dentinadhäsive, und v.Meerbeek, M. et al., Morphological aspects of the resin-dentin interdiffusion zone with different dentin adhesive systems, J. Dent. Res. 1992, 71, 1530-40, vergleichen 27 Dentinadhäsive miteinander. Die in diesen Publikationen beschriebenen Materialien enthalten neben Phosphorsäure Aminosäuren, Komplexbildner, anorganische oder organische Säuren, teilweise in Mischung mit Metallsalzen, zur Vorbehandlung der Zahnoberfläche.

Diese alternativen Konditionierungsmittel haben, je nach Art der Säure, weiterer Zusätze, Konzentration und Applikationsdauer teilweise positive Effekte auf die Dentinmorphologie. Sie führen jedoch im Vergleich zu Phosphorsäure meist zu einer schlechteren Schmelzkonditionierung, die einen weniger beständigen Haftverbund zwischen Adhäsiv und Schmelz zur Folge hat. Wie dem Fachmann geläufig geht dies in der Regel mit einer verminderten Randschlussqualität und einer verminderten Langlebigkeit der Versorgung einher. Zur Erzielung einer starken Schmelzhaftung und einer hohen Schmelzrandqualität stellt die Total-Etch-Technik unter Einsatz von Phosphorsäure daher nach wie vor das wirksamste Verfahren dar (Lopes, G.C. et al.; Enamel acid etching: a review, Compend. Contin. Educ. Dent. 2007, 28, 662-669).

Das Dokument EP 2 626 057 beschreibt eine wasserhaltige Lösung, die ein Zinnsalz enthält, zur therapeutischen Verwendung als Konditionierungsmittel für Zähne.

Der Erfindung liegt die Aufgabe zugrunde, Konditionierungsmittel für die Zahnbehandlung zur Verfügung zu stellen, die hinsichtlich Schmelzhaftung und Randschlussqualität mit dem Total-Etch-Verfahren unter Einsatz von Phosphorsäure vergleichbare Ergebnisse liefern, die aber nicht die oben geschilderten Nachteile dieses Verfahrens aufweisen. Insbesondere sollen die Mittel unabhängig vom Grad der Restfeuchte der Zahnoberfläche eine hohe Haftung ergeben und nicht den Abbau von Kollagen induzieren. Außerdem sollen sich die Mittel gleichzeitig auf Zahnschmelz und auf Dentin anwenden lassen, ohne dass unterschiedliche Einwirkungszeiten zu beachten währen. Schließlich sollen die Mittel nicht durch ein Überätzen von Dentin das Auftreten post-operativer Empfindlichkeiten hervorrufen. Erfindungsgemäß wird diese Aufgabe durch wasserhaltige Lösungen gelöst, die mindestens ein Salz von Zr⁴⁺, Hf⁴⁺, Ti⁴⁺ oder Bi³⁺ enthalten. Es wurde überraschend gefunden, dass sich solche Lösungen zur Verwendung als Konditionierungsmittel bei der dentalen Restaurationstherapie eignen.

Erfindungsgemäß wird unter einem Konditionierungsmittel für die dentale Restaurationstherapie ein Mittel verstanden, das direkt auf die Zahnoberfläche, d.h. auf den Zahnschmelz und/oder das Dentin, und insbesondere auf die nach mechanischer Bearbeitung des Zahns vorhandene Schmierschicht aufgetragen werden kann und das die Zahnoberfläche für den Auftrag nachfolgender dentaler Restaurationsmaterialien, wie z.B. von Adhäsiven, vorbereitet, indem es die Haftung dieser Materialien an Zahnschmelz und/oder Dentin verbessert.

Es wurde gefunden, dass wässrige Lösungen der genannten Übergangsmetall- und Hauptgruppenmetallsalze auch ohne den Zusatz von anorganischen oder organischen Säuren in der Lage sind, die Zahnhartsubstanz anzuätzen und die Schmierschicht zumindest teilweise aufzulösen.

Die Salze des Zr⁴⁺, Hf⁴⁺, Bi³⁺ und Ti⁴⁺ und insbesondere Salze des Zr⁴⁺, Hf⁴⁺ und Bi³⁺ können allein oder als Mischung von zwei oder mehreren dieser Salze eingesetzt werden.

Erfindungsgemäß sind in allen Fällen solche Metallsalzlösungen ganz besonders bevorzugt, die neben den Metallsalzen keine zugesetzte Säure enthalten, wobei unter Säuren Brönsted-Säuren, d.h. Protonen abspaltende Säuren mit einem pK-Wert <9 verstanden werden. Die erfindungsgemäßen Metallsalzlösungen enthalten damit vorzugsweise keine Mineralsäuren, keine Phosphorsäure und keine organischen Säuren wie Carbon-, Phosphon- und Sulfonsäuren.

Die erfindungsgemäß verwendeten Metallsalze sind vorzugsweise die Salze starker Säuren, insbesondere von Mineralsäuren, d.h. sie enthalten als Anionen vorzugsweise Cl⁻, Br⁻, I⁻, NO₃⁻ SO₄²⁻, ggf. als gemischte Salze mit Oxoanionen.

Allgemein sind solche Salze bevorzugt, die keine Anionen enthalten, welche die Bildung von Aqua-Komplexen oder das Erzielen homogener Lösungen im gewünschten Konzentrationsbereich behindern oder die eine puffernde Wirkung haben. Die erfindungsgemäßen Lösungen enthalten somit vorzugsweise kein EDTA, keine Nitrilotriessigsäure (NTA), kein Formiat, Acetat, Carbonat, Hydrogencarbonat und keine Salze von Säuren mit einem pKa-Wert > 3.

Erfindungsgemäß besonders geeignete Übergangs- und Hauptgruppenmetallsalzlösungen sind Lösungen von ZrOCl₂, ZrO(NO₃)₂, Zr(SO₄)₂, Hf(SO₄)₂, HfCl₄, Hf(NO₃)₄, TiOSO₄, Bi(NO₃)₃ oder Mischungen davon.

Die erfindungsgemäßen Übergangs- und Hauptgruppenmetallsalzlösungen können neben den oben genannten Metallsalzen vorzugsweise zusätzlich ein oder mehrere Salze anderer Hauptgruppen- und/oder Übergangsmetalle enthalten, wobei im Fall von Hauptgruppenmetallen Salze von Metallen mit einer Wertigkeit +2 bis +3 und im Fall von Übergangsmetallen Metalle mit einer Wertigkeit von +2 besonders bevorzugt sind. Ganz besonders bevorzugt sind Salze des Aluminiums, Kupfers oder des Zinks, insbesondere Salze des Cu²⁺, Zn²⁺ und Al³⁺, wie z.B. Al(NO₃)₃, CuSO₄ und ZnSO₄. Es wurde gefunden, dass diese Metalle die Wirksamkeit der Lösungen gegen MMPs verstärken.

Gemäß einer besonders bevorzugten Ausführungsform besteht die erfindungsgemäße Formulierung im Wesentlichen aus einer wässrigen oder wässrig-organischen Lösung eines oder mehrerer Salze von Übergangs- und Hauptgruppenmetallen und enthält keine Zusätze von Säuren, wie organischen oder anorganischen Säuren.

Es wurde gefunden, dass die erfindungsgemäß verwendeten Metallsalzlösungen in der Lage sind, Dentin und Zahnschmelz anzuätzen und die Schmierschicht ganz oder teilweise aufzulösen. Der genaue Grund für diese Wirkung ist nicht bekannt. Es wird angenommen, dass die Metallsalze in wässriger Lösung sogenannte Aquokomplexe bilden, d.h. Komplexe, die mindestens ein Wassermolekül als Ligand enthalten, und dass diese Komplexe durch die Abgabe von H⁺ entsprechend der Reaktionsgleichung (1) sauer reagieren:

Me(H₂O)ⁿ⁺ + H₂O ⇆ Me(OH)⁽ⁿ⁻¹⁾⁺ + H₃O⁺ (1)

Erfindungsgemäß sind daher Salze von solchen Übergangs- und Hauptgruppenmetallen bevorzugt, die Aquokomplexe bilden, insbesondere solche, die Aquokomplexe mit einem pKa-Wert in wässriger Lösung bei 25°C von -2 bis +4 (Bi³⁺, Hf⁴⁺, Zr⁴⁺, Ti⁴⁺), vorzugsweise -1 bis +2 (Bi³⁺, Hf⁴⁺, Zr⁴⁺) bilden (siehe S. J. Hawkes, Journal of Chemical Education, Vol. 73, No. 6, (1996), 516-517).

Das Metallkation Me bestimmt durch seine Ladungsdichte die Polarisierung des koordinierten Wassers und so die Acidität der Salzlösung. Die erfindungsgemäßen Übergangsmetallsalze enthalten Metallkationen mit hoher Ladung, d.h. drei- oder vierwertige Metallkationen (Me³⁺ bzw. Me⁴⁺).

Die in (1) abgespaltenen Protonen bewirken vermutlich die Auflösung des Hydroxylapatits von Zahnschmelz, Dentin und Schmierschicht entsprechend der Reaktionsgleichung (2):

Ca₅(PO₄)₃OH + H₃O⁺ ⇆ 5Ca²⁺ + 3PO₄³⁻ + 2H₂O (2)

Die freigesetzten Phosphatanionen können anschließend mit den Metallkationen des Übergangsmetallsalzes entsprechend der Reaktionsgleichung (3) Phosphate bilden (beispielhaft für Me = Zr⁴⁺):

3Zr(OH)³⁺ + 4PO₄³⁻ → Zr₃(PO₄)₄ + 3OH⁻ (3)

Erfindungsgemäß sind solche Übergangsmetallsalze bevorzugt, deren Metallkationen schwerlösliche Phosphate bilden, d.h. Phosphate, die unter den Anwendungsbedingungen ausfallen. Schwerlöslich bedeutet hier ein Löslichkeitsprodukt in Wasser bei 20°C von <10⁻⁴, bevorzugt von <10⁻⁶, besonders bevorzugt von <10⁻⁸.

Die Abwesenheit zugesetzter Säuren, insbesondere von Phosphorsäure oder mehrzähnigen organischen Säuren wie Oxal-, Malein- oder Zitronensäure, erhöht die Aktivität des Metallions und begünstigt so die Bildung schwerlöslicher Metallphosphate. Dies ist ein Grund dafür, dass die erfindungsgemäßen Lösungen bevorzugt keine zugesetzten Säuren oder Komplexbildner wie ED-TA enthalten.

Durch das Ausfällen der Phosphationen wird das Gleichgewicht gemäß Reaktionsgleichung (2) nach rechts verschoben und so die Auflösung des nur schwer zu ätzenden Zahnschmelzes begünstigt. Die Reaktion endet, wenn das Konditionierungsmittel verbraucht ist.

Trotz der guten Ätzwirkung auf Schmelz wurde überraschend gefunden, dass die erfindungsgemäß verwendeten Übergangs- und Hauptgruppenmetallsalzlösungen nicht zu einer Überätzung des Dentins führen. Es wird angenommen, dass die Übergangsmetallsalze durch Bindung an koordinierende Gruppen des Kollagens, wie z.B. -OH, -COOH, -CONH- oder -NH₂, zu einer Vernetzung der in der Schmierschicht vorhandenen Kollagentrümmer führen und so Regionen mit erhöhtem Proteingehalt (z.B. das durch Schmierschicht gefülltes Innenvolumen der Dentintubuli) weniger stark geätzt werden. Durch die lose anhaftende, mit dem Abspülen entfernte Metallsalz-Kollagenschicht wird die diffusionsgetriebene Reaktion des Ätzmittels mit der Mineralphase des Dentins verlangsamt. Dadurch wird ein Überätzen des empfindlicheren Dentins verhindert und das Risiko postoperativer Empfindlichkeiten deutlich reduziert, während auf dem kollagenfreien Zahnschmelz die gewünscht stärkere Ätzwirkung eintritt.

Auch im Hinblick auf die Kollagenvernetzung ist die Abwesenheit von anorganischen oder organischen Säuren und von Chelatbildnern von Vorteil, weil diese die Reaktion der Metallkationen mit den funktionellen Gruppen des Kollagens behindern.

Durch die Wahl der Metallsalze und deren Konzentrationen kann die Aktivität der erfindungsgemässen Lösung bei der Zahnkonditionierung entsprechend den Gleichungen (1) und (3) eingestellt werden.

Überraschenderweise führt das Ätzen von Dentin mit den erfindungsgemäßen Metallsalzlösungen zu einer merklich erhöhten Resistenz gegen das Übertrocknen. Im klinischen Gebrauch stellt dies eine sehr vorteilhafte Erhöhung der Techniktoleranz im Vergleich zur etablierten Phosphorsäurekonditionierung dar. Es wird vermutet, dass auch hierfür eine ionische Vernetzung der Kollagenfasern mit Übergangs- und Hauptgruppenmetallsalzen während des Ätzprozesses verantwortlich ist, was die Kollagenfasern gegen ein Kollabieren beim Übertrocknen stabilisiert.

Weiterhin wurde gefunden, dass die erfindungsgemäßen Übergangs- und Hauptgruppenmetallsalzlösungen nicht zu einer Aktivierung von Matrixmetalloproteinasen oder Cysteincathepsinen führen. Der Einsatz der Metallsalzlösungen reduziert vielmehr die MMP-Aktivität, und erhöht so die Langzeitbeständigkeit des Dentinverbundes. Auch dies ist vermutlich auf eine Wechselwirkung der Übergangs- und Hauptgruppenmetallsalze mit Dentinproteinen zurückzuführen. Die Anbindung von Metallionen und insbesondere von polyvalenten Metallionen an Proteine wie Kollagen oder kollagengetragenen Enzyme kann durch die eingebrachte Ladung eine dauerhafte Veränderung deren Tertiärstruktur bewirken. Bei Matrixmetalloproteinasen oder Cysteincathepsinen führt insbesondere der Kontakt mit den erfindungsgemäß bevorzugten polyvalenten Metallsalzen jedenfalls zu einer drastischen Senkung oder sogar einem vollständigen Verlust der Aktivität, was auf eine Veränderung der Tertiärstruktur zurückgeführt wird.

Die erfindungsgemäß verwendeten Metallsalze und insbesondere die erfindungsgemäß bevorzugten Metallsalze sind gut biologisch verträglich und weisen keine Eigenfärbung auf. Sie bilden keine farbigen Niederschläge auf dem Zahn, wie dies bei Chrom-, Nickel-, Eisen-, Osmium-, Vanadium- oder Silbersalzen oft der Fall ist. Zudem sind die Salze in Lösemitteln oder Lösungsmittelgemischen, die zur intraoralen Anwendung geeignet sind, ausreichend gut löslich.

Erfindungsgemäß sind Lösungen bevorzugt, die 0,5 bis 25 Gew.-%, besonders bevorzugt 2 bis 15 Gew.-% und ganz besonders bevorzugt 4 bis 10 Gew.-% Übergangs- und/oder Hauptgruppenmetallsalz(e) enthalten, insbesondere ZrOCl₂, ZrO(NO₃)₂, Zr(SO₄)₂, Hf(SO₄)₂, HfCl₄, Hf(NO₃)₄, TiOSO₄, Bi(NO₃)₃ oder Mischungen davon.

Die als mögliche Zusätze genannten Metallsalze insbesondere der Metalle Cu²⁺, Zn²⁺ und Al³⁺ werden vorzugsweise in Konzentrationen von 0.01% bis 5% eingebracht.

Darüber hinaus enthalten die erfindungsgemäßen Lösungen mindestens ein Lösungsmittel, d.h. Wasser oder eine Mischung von Wasser und organischem Lösungsmittel. Die Zugabe von einem oder mehreren organischen Lösungsmitteln kann vorteilhaft sein, z.B. um die Oberflächenbenetzung, die Stabilität der Formulierung und/oder die Abspülbarkeit zu verbessern. Bevorzugte organische Lösungsmittel sind Glycerin, Ethanol, Ethylengylkol, Propylenglykol, Propandiol, Butandiol, Polyethylenglykol und Mischungen davon, insbesondere Glycerin.

Besonders bevorzugt sind Lösungen, die 2 bis 15 Gew.-% Übergangs- und/oder Hauptgruppenmetallsalz, 36 bis 98 Gew.-% Wasser und 0 bis 49 Gew.-% organisches Lösungsmittel enthalten, oder Lösungen die 4 bis 10 Gew.-% Übergangs- und/oder Hauptgruppenmetallsalz, 42 bis 96 Gew.-% Wasser und 0 bis 48 Gew.-% organisches Lösungsmittel enthalten.

Die erfindungsgemäßen Ätzmittel können außerdem vorteilhaft ein oder mehrere Additive enthalten, wie Verdickungsmittel, Farbstoffe, antibakterielle Mittel, Konservierungsmittel, Geschmacksstoffe, Duftstoffe und Verlaufshilfen.

Verdickungsmittel dienen der Einstellung einer gut handhabbaren Konsistenz, die eine tropffreie und punktgenaue Applikation auf dem Zahn ermöglicht. Verdickungsmittel können aus anorganischen oder organischen Materialien ausgewählt werden. Bevorzugte anorganische Materialien sind nanopartikuläre pyrogene Kieselsäuren, wie z.B. Aerosile. Bevorzugte organische Materialien sind Cellulose-Derivate, Assoziativverdicker auf Polyurethanbasis und Polyethylenoxid. Mischungen von organischen und anorganischen Verdickern können ebenfalls verwendet werden. Bevorzugte Verdickungsmittel sind Polyethylenoxide, Kieselsäuren und insbesondere Mischungen davon.

Farbstoffe dienen der leichten Erkennbarkeit des Ätzmittels auf dem Zahn, um die behandelte Fläche eingrenzen zu können und eine vollständige Entfernung des Ätzmittels beim Abspülen zu gewährleisten. Die Farbmittel müssen sich beim Abspülen des Ätzmittels leicht und vollständig entfernen lassen. Farbstoffe können aus anorganischen oder organischen Verbindungen bestehen. Als anorganische Farbstoffe kommen insbesondere Pigmente, z.B. Cobaltspinelle, in Frage. Bevorzugte organische Farbstoffe sind lösliche Verbindungen, wie z.B. Methylenblau, und dispergierbare Pigmente, wie z.B. Kupfer-Phthalocyanin.

Die Additive werden ggf. in einer Menge zugesetzt, die zur Erzielung des gewünschten Effekts, also z.B. zur Einstellung einer bestimmten Viskosität oder Farbe, erforderlich ist.

Die erfindungsgemäßen Dentalwerkstoffe eignen sich primär zur Konditionierung der Oberfläche natürlicher Zähne (Dentin und Zahnschmelz). Es handelt sich hierbei um eine therapeutische Anwendung, die typischerweise intraoral durch den Zahnarzt erfolgt. Beispielhafte Anwendungen sind das Konditionieren der Zahnoberfläche vor dem Aufbringen dentaler Zemente, Füllungskomposite, Beschichtungsmaterialien und Verblendmaterialien, insbesondere die Konditionierung der Zahnoberfläche bei der dentalen Restaurationstherapie, d.h. bei der Restauration geschädigter Zähne, z.B. bei der restaurativen Füllungstherapie.

Die Anwendung der erfindungsgemäßen Lösungen erfolgt vergleichbar zu bekannten Ätzmitteln auf Phosphorsäurebasis.

Die **restaurative Füllungstherapie** umfasst vorzugsweise die folgenden Verfahrensschritte:
(1) Präparation der kariösen Läsion durch mechanische Exkavation des demineralisierten Gewebes.
(2) Auftragen des erfindungsgemäßen Konditionierungsmittels auf die zu verklebenden Zahnoberflächen, z.B. mit einem pinselartigen Dentalapplikator ("Microbrush") oder, im Falle pastöser Formulierungen, mit einer Spritze. Das Konditionierungsmittel wird hierbei lediglich auf den Zahn appliziert, ein Bewegen des Mittels während der Einwirkzeit ist nicht nötig.
(3) Nach der vorgesehenen Einwirkzeit (5-120s, bevorzugt 10-90s, besonders bevorzugt 15-60s) wird das Konditionierungsmittel mit einem Wasserstrahl von der Zahnoberfläche abgespült. Eine Unterscheidung der Einwirkdauer für Schmelz und Dentin zur Vermeidung eines Überätzens des Dentins ist, anders als bei Phosphorsäure, nicht notwendig. Auch ist die Einwirkdauer auf Dentin, anders als bei Phosphorsäure, nicht streng zu begrenzen, um ein Überätzen zu vermeiden. Bevorzugt ist eine gleich lange Einwirkdauer auf Schmelz und Dentin, da dies im klinischen Alltag am einfachsten zu verwirklichen ist.
(4) Die Zahnoberfläche wird mit einem Luftstrom so lange getrocknet, bis der geätzte Schmelz ein kreidig-weißes Aussehen annimmt. Bei diesem Arbeitsschritt kommt es auf Dentin, das mit Phosphorsäure geätzt wurde, häufig zum Übertrocknen, und so zum Kollaps der Kollagenfibrillen. Daher wird beim konventionellen Vorgehen nach dem Trockenblasen das Dentin häufig selektiv z.B. mit einem feuchten Microbrush wieder befeuchtet. Bei Einsatz des erfindungsgemäßen Ätzmittels ist ein Wiederbefeuchten des Dentins nach dem Trockenblasen nicht notwendig.
(5) Auf die geätzte Zahnoberfläche wird ein Dentaladhäsiv aufgetragen.
(6) Das Adhäsiv wird mit einem Luftstrom verblasen und dann z.B. mit Licht gehärtet.
(7) Ein für das Adhäsiv und den Füllungstyp geeignetes Füllungskomposit wird in die Kavität eingebracht und lichtgehärtet.

Als Adhäsiv kann jedes handelsübliche Dentaladhäsiv eingesetzt werden. Die Anwendung erfolgt gemäß Gebrauchsinformation. Dieses Verfahren ist ebenfalls Gegenstand der Erfindung.

Die erfindungsgemäßen Ätzmittel eignen sich für alle dentalen Anwendungen, in denen üblicherweise Phosphorsäure als Ätzmittel eingesetzt wird. Weitere therapeutische Anwendungen, in denen die erfindungsgemäßen Metallsalzlösungen vorteilhaft als Konditionierungsmittel angewendet werden können, sind:
- Die Befestigung **festsitzender Prothetik** durch adhäsive Zementierung vorgefertigter metallischer, keramischer oder polymerer Restaurationen wie Kronen, Brücken, Inlays, Onlays. Hierbei wird wie oben beschrieben vorgegangen, aber nach Abschluss des Adhäsivbehandlung (Konditionierung) mittels eines dünnflüssigen Zements oder Befestigungskomposits eine Restauration auf dem Zahn befestigt. Die Härtung des Befestigungskomposits erfolgt auf übliche Weise, z.B. durch Selbst- oder Lichthärtung.
- **Präventive Maßnahmen**, wie der Versiegelung von Zahnfissuren. Bei der Versieglung okklusaler Zahnfissuren wird die Metallsalzlösung auf den Zahn aufgebracht und nach dem Abspülen des Ätzmittels und Trockenblasen der Zahnoberfläche ein fließfähiger Fissurenversiegler, z.B. ein methacrylatbasierendes fließfähiges Kompositmaterial, aufgetragen und anschließend ausgehärtet.
- **Kieferorthopädische Maßnahmen**, wie der Befestigung von Brackets oder Zahnspangen. Hier wird die Metallsalzlösung auf die betreffende Stelle des Zahns aufgebracht und nach dem Abspülen des Ätzmittels und Trockenblasen der Zahnoberfläche ein spezieller Befestigungskomposit eingesetzt, mit dem eine metallische oder keramische Drahtaufnahme auf dem Zahn befestigt wird. Der Befestigungskomposit wird anschließend ausgehärtet.

Die erfindungsgemäßen Metallsalzlösungen können auch extra-oral, d.h. nicht therapeutisch, eingesetzt werden, beispielsweise zur Vorbehandlung der Oberfläche von Dentalrestaurationen wie Prothesen, künstlichen Zähnen, Inlays, Onlays, Kronen und Brücken. Diese Vorhandlung dient z.B. zur Verbesserung der Haftung bei der adhäsiven Befestigung und/oder zur Reinigung der Oberflächen, z.B. durch das Entfernen proteinöser Verunreinigungen insbesondere von glaskeramischen Restaurationen.

Im Folgenden wird die Erfindung anhand von Figuren und Ausführungsbeispielen näher erläutert.
Figur 1 zeigt eine demineralisierte Zahnscheibe unmittelbar nach der Präparation, nach 24stündiger und nach 48stündiger Lagerung. Nach der Demineralisierung mit Säure bleibt die Kollagenmatrix des Zahns zurück. Diese hat sich während der Lagerung nicht verändert, d.h. das Kollagen wurde durch die Demineralisierung nicht abgebaut.
Figur 2 zeigt eine demineralisierte Zahnscheibe nach Zugabe von Typ I-Kollagenase. Die Kollagenase bewirkt innerhalb von 48h einen fast vollständigen Abbau der Kollagenmatrix.
Figur 3 zeigt eine demineralisierte Zahnscheibe, die vor der Kollagenasebehandlung für 60s in eine 2%ige Zirkonylchlorid-(ZrOCl₂) Lösung getaucht wurde. Fig. 3 zeigt, dass durch das Zirkoniumsalz der Abbau der Kollagenmatrix praktisch vollständig inhibiert wird.
Figur 4 zeigt eine demineralisierte Zahnscheibe, die in 1%ige Zirkonylchlorid-Lösung getaucht wurde. Auch hier wird der Abbau der Kollagenmatrix vollständig inhibiert.
Figur 5 zeigt das Ätzmuster auf Rinderzahnschmelz und Rinderdentin nach Ätzen mit 37%iger Phosphorsäure für 30s bzw. 15s.
Figur 6 zeigt das Ätzmuster auf Rinderzahnschmelz und Rinderdentin nach Ätzen mit einer 4%igen Lösung von ZrOCl₂ in einer 1:1 Mischung aus Wasser und Glycerin (**Lösung A**) für 30s bzw. 15s.
Figur 7 zeigt das Ätzmuster auf Rinderzahnschmelz und Rinderdentin nach Ätzen mit einer 4%igen Lösung von Zr(SO₄)₂ in einer 1:1 Mischung aus Wasser und Glycerin (**Lösung B**) für 30s bzw. 15s.
Figur 8 zeigt das Ätzmuster auf Rinderzahnschmelz und Rinderdentin nach Ätzen mit einer 4%igen Lösung von ZrO(NO₃)₂ in einer 1:1 Mischung aus Wasser und Glycerin (**Lösung D**) für 15s.
Figur 9 zeigt das Ätzmuster auf Rinderzahnschmelz und Rinderdentin nach Ätzen mit einer 6%igen Lösung von ZrO(NO₃)₂ in Wasser (**Lösung E**) für 15s.
Figur 10 zeigt das Ätzmuster auf Rinderzahnschmelz und Rinderdentin nach Ätzen mit einer 10%igen Lösung von ZrO(NO₃)₂ in einer 1:1 Mischung aus Wasser und Glycerin (**Lösung F**) für 15s.
Figur 11 zeigt das Ätzmuster auf Rinderzahnschmelz und Rinderdentin nach Ätzen mit einer 15%igen Lösung von ZrO(NO₃)₂ in einer 1:1 Mischung aus Wasser und Glycerin (**Lösung G**) für 15s.
Figur 12 zeigt das Ätzmuster auf Rinderzahnschmelz und Rinderdentin nach Ätzen mit einer 10%igen Lösung von ZrO(NO₃)₂ in einer 1:1 Mischung aus Wasser und PEG-400 (**Lösung H**) für 15s.
Figur 13 zeigt das Ätzmuster auf Rinderzahnschmelz und Rinderdentin nach Ätzen mit einer 10%igen Lösung von ZrO(NO₃)₂ in einer Mischung aus Wasser, Glycerin, Verdicker und Pigment (**Lösung I**) für 15s.
Figur 14 zeigt das Ätzmuster auf Rinderzahnschmelz und Rinderdentin nach Ätzen mit einer 0.5%igen Lösung von TiOSO₄ in einer Mischung aus Wasser und Glycerin (**Lösung J**) für 30s bzw. 15s.
Figur 15 zeigt das Ätzmuster auf Rinderzahnschmelz und Rinderdentin nach Ätzen mit einer 2%igen Lösung von Bi(NO₃)₃ in Wasser (**Lösung L**) für 30s bzw. 15s.
Figur 16 zeigt das Ätzmuster auf Rinderzahnschmelz und Rinderdentin nach Ätzen mit einer 4%igen Lösung von Hf(SO₄)₂ in Wasser (**Lösung M**) für 30s bzw. 15s.
Figur 17 zeigt das Ätzmuster auf Rinderzahnschmelz und Rinderdentin nach Ätzen mit einer 4%igen Lösung von HfCl₄ in Wasser (**Lösung N**) für 30s bzw. 15s.
Figur 18 zeigt das Ätzmuster auf Rinderzahnschmelz und Rinderdentin nach Ätzen mit einer Lösung enthaltend 6% ZrO(NO₃)₂ und 4% ZnSO₄ in Wasser (**Lösung O**) für 30s bzw. 15s.
Figur 19 zeigt das Ätzmuster auf Rinderzahnschmelz und Rinderdentin nach Ätzen mit einer Lösung enthaltend 6% ZrO(NO₃)₂ und 4% CuSO₄ in Wasser **(Lösung P)** für 30s bzw. 15s.
Figur 20 zeigt das Ätzmuster auf Rinderzahnschmelz und Rinderdentin nach Ätzen mit einer Lösung enthaltend 6% ZrO(NO₃)₂ und 4% Al₂(SO₄)₃ in Wasser (**Lösung Q**) für 30s bzw. 15s.

### Ausführungsbeispiele

### Beispiel 1

### Herstellung von Konditionierungsmitteln

Durch Mischen der Komponenten wurden Übergangsmetalllösungen mit der in Tabelle 1 angegebenen Zusammensetzung hergestellt.

**Tabelle 1: Zusammensetzung der Konditionierungsmittel**

| **Lösung** | **Zusammensetzung (Gew.-%)** | | |
|---|---|---|---|
| | **Metallsalz** | **Lösemittel** | **Verdicker/Additiv** |
| **A** | ZrOCl₂ (4%) | H₂O (48%) Glycerin (48%) | - |
| **B** | Zr(SO₄)₂ (4%) | H₂O (48%) Glycerin (48%) | - |
| **C** | Zr(SO₄)₂ (4%) | H₂O (96%) | - |
| **D** | ZrO (NO₃)₂ (4%) | H₂O (48%) Glycerin (48%) | - |
| **E** | ZrO(NO₃)₂ (6%) | H₂O (47%) Glycerin (47%) | - |
| **F** | ZrO(NO₃)₂ (10%) | H₂O (45%) Glycerin (45%) | - |
| **G** | ZrO(NO₃)₃ (15%) | H₂O (42.5%) Glycerin (42.5%) | - |
| **H** | ZrO(NO₃)₃ (10%) | H₂O (45%) PEG-400 (45%) | - |
| **I** | ZrO(NO₃)₂ (10%) | H₂O (42.75%) Glycerin (42.75%) | Hydroxyethylcellulose (Cellosize QP-30000H)(2%) Polyurethansystem (Tafigel Pur 61) (2%) Pigment (Sicopal Blau K6210) (0.5%) |
| **J** | TiOSO₄ (0.5%) | H₂O (49.75%) Glycerin (49.75%) | - |
| **L** | Bi(NO₃)₃ (2%) | H₂O (98%) | - |
| **M** | Hf (SO₄)₂ (4%) | H₂O (96%) | - |
| **N** | HfCl₄ (4%) | H₂O (96%) | - |
| **O** | 6% ZrO (NO₃) ₂ | H₂O (90%) | - |
| | 4% ZnSO₄ | | |
| **P** | 6% ZrO (NO₃) ₂ | H₂O (90%) | - |
| | 4% CuSO₄ | | |
| **Q** | 6% ZrO (NO₃) ₂ | H₂O (90%) | - |
| | 4% Al₂(SO₄)₃ | | |

### Beispiel 2

### Bestimmung der Dentinhaftung

Die Bestimmung der Haftung an Zahnhartsubstanz erfolgte in Anlehnung an das Ultradent-Protokoll (ISO 29022). Hierzu wurden die erfindungsgemäßen Lösungen A-Q jeweils auf die präparierte Zahnoberfläche aufgebracht. Als Testsubstrat dienten Rinderfrontzähne, die nach Entfernen der Pulpa und des Zahnhalses bis zur Schmelz-Dentin-Grenze in ein kalthärtendes Polyester-Styrol-Harz eingebettet wurden. Unmittelbar vor der Herstellung der Probekörper wurde der eingebettete Zahn mit P120 SiC-Schleifpapier unter Wasserkühlung mit einer Schleifmaschine plan-parallel bzgl. der Zylinderstirnseiten aufgeschliffen. Durch regelmäßige Sichtkontrolle wurde sichergestellt, dass die Dentinschicht freigelegt wurde. Anschließend wurde die Probe mit P400 SiC-Schleifpapier so lange beschliffen, bis keine Spuren des P120 Schleifschrittes mehr sichtbar sind. Die mit P400 Papier beschliffenen Zahnoberflächen wurden dann unter fließendem handwarmem Wasser (25-35°C) für ca. 10s je Zahn ohne weitere mechanische Einwirkung abgespült. Der Zahn wurde danach bis zur Adhäsivapplikation in auf 23-37°C temperiertem Leitungswasser gelagert.

Die Zähne wurden dann mit Druckluft trockengeblasen, bis kein Wasser mehr auf der Oberfläche sichtbar war. Das Ätzmittel wurde mit einem pinselartigen Dentalapplikator ("Microbrush") aufgetragen. Im Fall pastöser Formulierungen wurde das Konditionierungsmittel mit einer Spritze appliziert. Die zu verklebende Zahnoberfläche wurde mit einer durchgehenden Schicht des Konditionierungsmittels bedeckt. Die Schicht wurde für die vorgesehene Einwirkdauer ohne Agitation auf dem Zahn ruhen gelassen. Nach einer Einwirkzeit von 15 bis 30s wurde das Konditionierungsmittel mit dem Wasserstrahl einer Dentaleinheit solange abgespült, bis keine Rückstände mehr sichtbar waren. Nach dem Abspülen wurde die geätzte Dentinoberfläche nach dem in Tabelle 2 angegebenen Verfahren getrocknet.

Danach wurden zwei Tropfen des jeweiligen Adhäsivs auf einer Mischplatte vorgelegt und mit einem Microbrush auf die vorbehandelte Zahnoberfläche aufgebracht. Die Anwendung des Adhäsivs erfolgte gemäß der Gebrauchsinformation des Herstellers wie unten beschrieben. Das Adhäsiv wurde anschließend mit einem anfänglich schwachen, dann stärker werdenden Luftstrom aus ca. 2-8 cm Entfernung so lange verblasen, bis ein unter dem starken Luftstrom unbeweglicher Film entstanden war. Diese Adhäsivschicht wurde dann mit einem Lichtpolymerisationsgerät (Bluephase Style; Ivoclar Vivadent) für 10s gehärtet.

Der so vorbereiteten Zähne wurden mit der präparierten Seite nach oben in die Ultradent Applikationsvorrichtung eingebracht und durch leichtes Anziehen der Halterungsschrauben in der Applikationsform fixiert. Anschließend wurde durch die Öffnung der Applikationsform der einzusetzende Füllungskomposit (Tetric EvoCeram BulkFill; Ivoclar Vivadent) in einer Schichtstärke von 1 - 1.5 mm appliziert und dann für 20s mittels Belichtung durch eine Bluephase Style ausgehärtet. Nach erfolgter Polymerisation konnten die Halteschrauben gelöst und der Verbundprüfkörper durch sanften Druck mit einem Kompositstopfer auf den Kompositpropf aus der Einspannvorrichtung entfernt werden. Pro Messserie wurden 5 Probenkörper hergestellt und vor der Messung der Haftung für 24h in Leitungswasser bei 37 ± 1°C gelagert.

Vor der eigentlichen Haftungsmessung wurde der Durchmesser des Komposit-Pfropfes mit einer Schieblehre auf zwei Nachkommastellen genau gemessen und dieser Wert in der Auswertesoftware der Prüfmaschine hinterlegt (testXpert; Fa. ZWICK-ROELL).

Für die Bestimmung der Haftkraft wurde die Universaltestmaschine ZWICK 010 (Fa. ZWICK-ROELL) eingesetzt. Der Probekörper wurde mit der Schneidefläche nach unten ("crown down") in der für ISO 29022 vorgesehenen Einspannvorrichtung fixiert und anschließend eine Abschervorrichtung so positioniert, dass deren halbrunde Ausformung den Kompositpfropf exakt erfasste und die Abschervorrichtung plan am Zahn anlag. Durch Starten der Messprozedur wurde die Abschervorrichtung mit 1mm/min abgesenkt und der Prüfkörper dadurch parallel zur Substratoberfläche mit einer Schubspannung bis zum Bruch belastet. Die Haftfestigkeit errechnet sich als der Quotient aus der beim Bruch anliegenden Bruchkraft und der Haftfläche unter dem Kompositpfropf. Die Angabe erfolgt in Megapascal (MPa).

Die Haftwertuntersuchungen umfassten neben den absoluten Haftwerten, auch die Bruchart bei Versagen der Probekörper. Probekörper versagen bei Scherbelastung entweder "kohäsiv" im Dentin, oder "adhäsiv" zwischen Dentin und Adhäsiv. Ein kohäsiver Bruch erfolgt bei Messungen auf Dentin nach ISO 29022 üblicherweise bei Haftwerten >20 MPa und deutet auf einen direkten Kontakt zwischen intakter Zahnhartsubstanz und Adhäsiv hin. Adhäsive Brüche hingegen erfolgen meist bei Haftwerte <20 MPa und deuten auf eine vom Adhäsiv nicht durchdringbare Trennschicht (z.B. kollabiertes Kollagen) auf der intakten Zahnhartsubstanz hin.

Um die Techniktoleranz der erfindungsgemäßen Konditionierungslösungen gegenüber der etablierten Phosphorsäure zu prüfen, wurde zur Haftwertuntersuchungen in einigen Fällen die Feuchtigkeit der Dentinoberfläche vor der Adhäsivapplikation durch unterschiedlich langes Trocknen variiert. Folgende Feuchtigkeitsgrade wurden erzeugt (Tabelle 2):

**Tabelle 2: Trocknungsgrade (Dentin)**

| **Feuchtigkeitsgrad** | **Trocknungsbedingungen** |
|---|---|
| **Feucht** | Abtupfen mit feuchten Papiertüchern (Precision Wipes; Fa. KimWip) |
| **Trocken** | 5s starker Luftstrom (4bar) |
| **Sehr trocken** | 10 - 15s starker Luftstrom (4bar) |

Die Scherhaftwerte wurden gemäß der genannten ISO-Norm mit den kommerziellen Dentaladhäsiven Excite F, (Ivoclar Vivadent AG) und Prime & Bond NT (Dentsply) bestimmt. Die gewählten Adhäsive sind als Total Etch Adhäsive zur Anwendung auf mit Phosphorsäure geätzten Zahnoberflächen gedacht und weisen die zwei für diese Produkte üblichen Lösemittel auf (ExciTE F: Ethanol, Prime&Bond NT: Aceton). Die Anwendung beider Adhäsive auf geätzten Zahnoberflächen erfolgte gemäß den vom Hersteller vorgesehenen Gebrauchsinformationen.

**ExciTE F:** Eine Schicht des Adhäsivs wurde mit einem Microbrush auf die geätzte Zahnoberfläche aufgetragen, für 10s mit sanftem Druck einmassiert und dann mit einem Luftbläser so lange belüftet bis ein unbeweglicher Film entstanden war. Dieser wurde mit einem Lichtpolymerisationsgerät (Bluephase Style; Ivoclar Vivadent) für 10s belichtet. Anschließend wurde wie beschrieben ein Kompositpfropf (Tetric EvoCeram BulkFill; Ivoclar Vivadent) aufpolymerisiert und die Scherhaftung gemessen.

**Prime & Bond NT:** Eine durchgehende Schicht des Adhäsivs wurde per Microbrush auf die geätzte Zahnoberfläche aufgetragen. Sollte die Zahnoberfläche nach einer Einwirkauer von 20 s nicht mehr feucht glänzen, so wurde gemäß Gebrauchsinformation eine weitere Schicht Adhäsiv aufgetragen. Anschließend wurde mit einem Luftbläser so lange belüftet, bis ein durchgehender, glänzender Film entstanden war. Dieser wurde mit einem Lichtpolymerisationsgerät (Bluephase Style; Ivoclar Vivadent) für 10s belichtet. Danach wurde wie beschrieben ein Kompositpfropf (Tetric EvoCeram BulkFill; Ivoclar Vivadent) aufpolymerisiert und die Scherhaftung gemessen. Die Ergebnisse sind in Tabelle 3 zusammengefasst.

**Tabelle 3: Scherhaftwerte auf Dentin (24h@37°C)**

| **Lösung** | **Adhäsiv** | **Haftwerte in MPa (Bruchbild)** | | |
|---|---|---|---|---|
| | | **Feucht** | **Trocken** | **Sehr trocken** |
| EP¹ | | 28.7±2.8(4/5) | 12.9±4.8(0/5) | 8.3±2.2(0/5) |
| A | | 31.5±5.2(5/5) | 27.9±5.0(4/5) | 22.5±4.8(4/5) |
| B | | 28.7±5.7(5/5) | 25.8±8.8(3/5) | 27.2±13.0(3/5) |
| C | | - | 28.8±3.1(5/5) | - |
| D | | - | 22.2±8.7(4/5) | - |
| E | | 35.8±9.2(5/5) | 31.2±6.0(5/5) | 26.7±5.8(4/5) |
| F | | 27.5±6.1(5/5) | 30.4±6.9(3/5) | - |
| G | | 23.9±4.4(5/5) | 32.7±4.9(5/5) | - |
| H | Excite F | 30.6±5.4(5/5) | - | - |
| I | | 31.0±5.6(5/5) | 29.8±4.3(5/5) | - |
| J | | 22.2±4.4(5/5) | - | - |
| L | | 33.6±5.8(5/5) | - | - |
| M | | 32.5±6.7(5/5) | - | - |
| N | | 37.9±4.1(5/5) | - | - |
| O | | 29.2±7.3(5/5) | - | - |
| P | | 28.5±2.0(5/5) | - | - |
| Q | | 30.7±9.3(5/5) | - | - |
| EP¹ | Prime& Bond NT | 30.7±3.5(5/5) | 9.90±3.4(0/5) | - |
| F | | 33.2±5.7(5/5) | 23.6±3.2(4/5) | - |
| I | | 34.6±5.1(5/5) | 25.5±4.1(3/5) | - |

| | | | | |
|---|---|---|---|---|
| ¹ Email Preparator Blau, Fa. Ivoclar Vivadent: 37%ige Phosphorsäure (Vergleichsbeispiel) | | | | |

Die Daten in Tabelle 3 zeigen für die Konditionierungsmittel A-B und E-Q bei Einsatz auf feuchtem Dentin eine der konventionellen Phosphorsäure äquivalente Wirkung und unabhängig vom Adhäsiv adäquate Haftergebnisse. Wurde das Dentin stärker getrocknet oder gar übertrocknet, nahmen die Haftwerte der mit Phosphorsäure behandelten Oberflächen wie erwartet deutlich ab. Die als repräsentative Vertreter diesbezüglich untersuchten Konditionierungsmittel A-G und I wurden durch den Trockenprozess hingegen wenig bis gar nicht beeinflusst.

### Beispiel 3

### Bestimmung der Haftung an Zahnschmelz

Die Messung der Haftung an Zahnschmelz erfolgte analog zu Beispiel 2. Da Schmelz als nahezu rein anorganisches Gewebe keine Kollagenfibrillen enthält, wurde die Trocknungsdauer nicht variiert. Zum Test der Schmelzhaftung wurde daher nur trockenes Substrat untersucht, nach dem Abspülen des Ätzmittels wurde die Oberfläche ca. 5s mit einem starken Luftstrom aus einer Dentaleinheit getrocknet. Ziel war es, das dem Zahnarzt geläufige Bild einer kreidig-weißen Schmelzoberfläche zu erhalten. Um die Auswirkung einer gegenüber dem etablierten Standard von 30s auf 15s reduzierten Ätzdauer zu prüfen, wurden für ausgewählte Proben beide Einwirkperioden untersucht. Die Ergebnisse für die Schmelzhaftwerte sind in Tabelle 4 aufgeführt.

**Tabelle 4: Scherhaftwerte auf Zahnschmelz (24h@37°C)**

| **Lösung** | **Adhäsiv** | **30s Äztzeit (MPa)** | **15s Ätzzeit (MPa)** |
|---|---|---|---|
| EP¹ | | 29.6±4.7 | 27.2±8.8 |
| A | | 25.8±2.6 | 30.7±4.6 |
| B | | 24.8±5.8 | 33.5±6.1 |
| D | | - | 29.2±4.9 |
| E | | - | 28.8±2.5 |
| F | | - | 24.1±6.3 |
| G | | - | 23.5±8.5 |
| H | Excite F | - | 25.2±6.0 |
| I | | - | 28.3±1.9 |
| J | | 20.9±3.8 | - |
| L | | 26.2±1.3 | - |
| M | | 30.8±6.8 | - |
| N | | 26.7±6.4 | - |
| O | | 30.0±3.6 | - |
| P | | 20.7±2.5 | - |
| Q | | 27.2±7.3 | - |
| EP¹ | Prime & Bond NT | 24.5±6.5 | 17.8±2.8 |
| F | | 21.6±3.4 | 28.2±8.2 |
| I | | 31.0±4.2 | 25.5±6.6 |

| | | | |
|---|---|---|---|
| ¹ Email Preparator Blau, Fa. Ivoclar Vivadent: 37%ige Phosphorsäure (Vergleichsbeispiel) | | | |

Die in Tabelle 4 gezeigten Schmelzhaftwerte sind unabhängig vom getesteten Adhäsiv für die Konditionierungsmittel A - Q im Rahmen der Messgenauigkeit vergleichbar zur konventionellen Phosphorsäure. Auch durch Schwankungen in der Zahnsubstratbeschaffenheit niedriger erscheinende Werte sind hoch genug, um einen klinisch adäquaten Verbund zu sichern. Schmelzproben versagen wegen der Härte des Substrates stets adhäsiv, so dass die Versagensart (Bruchbild) hier nicht notiert wurde.

### Beispiel 4

### Bestimmung der Randqualität an Zahnschmelz

Es wurde die für die Bewertung des Schmelzverbundes wichtige Randqualität gemessen. Mit der Randqualität wird der fehlerfreie Anteil der Grenze zwischen Komposit und Zahn in einer kreisförmigen Kavität in Rinderzähnen bezeichnet. Sind z.B. auf 60% der Zahn-Komposit-Grenze Defekte wie Risse, Aufwölbungen oder Spalten zu bemerken, so beträgt die Randqualität 40%. Die Auswertung erfolgte per Rasterelektronenmikroskop (REM) "VP DSM" (Fa. Zeiss, Deutschland).

Zur Bestimmung der Randqualität wurden pro Messserie 5 Probekörper hergestellt. Die Präparation der Kavität erfolgte jeweils mit einem Diamantschleifkörper bei 40.000 U/min unter Wasserkühlung (Durchmesser: 4 mm, Tiefe: 2,5 mm) . Dazu diente ein Diamantschleifkörper Komet Nr. 909/040 (4 mm) für die erste Bohrung und ein zylindrischer Diamantschleifkörper Nr. 2504 (1,9 mm, 25µm) zum Finieren. Das Ätzmittel (Lösung B) wurde per Microbrush deckend auf die Kavitätentwände gebracht und 15s ohne Agitation einwirken gelassen. Anschließend wurde das Ätzmittel mit einem Wasserstrahl abgespült und die Kavitätenoberfläche mit einem Luftbläser trockengeblasen bis kreidigweißer Schmelz sichtbar wurde. Auf die getrocknete Oberfläche wurde das Adhäsiv ExciTE F (Ivoclar Vivadent) gemäß Gebrauchsinformation appliziert, einwirken gelassen, trockengeblasen und lichtgehärtet (Bluephase Style; Ivoclar Vivadent). In die so vorbereitete Kavität wurde dann der Füllungskomposit (Tetric EvoCeram; Fa. Ivoclar Vivadent) in 2mm Inkrementen eingebracht und jedes Inkrement vor dem nächsten Inkrement lichtgehärtet. Abschließend wurde die gelegte Füllung poliert (SiC-Papier, P600 grit, 1200 grit, 2500 grit, 0,5µm Masterprep (Buehler) / Polierscheibe (4000 grit)) und für 48 h in destilliertem Wasser bei 37°C gelagert.

Zur Vermeidung von Vakuumartefakten bei der anschließenden rasterelektronenmikroskopischen (REM) Untersuchung erfolgte die Prüfung an Replikas der Prüfkörper (Abdrucksilikon: "Virtual extra light body", fast set; Drucktopf; 2bar). Diese Abdrücke wurden nach Aushärtung mit Epoxidharz (Stycast) ausgegossen, um Blasenbildung durch das Ausdampfen des Silikons im Vakuum zu vermeiden. Auf diese Replikas wurde zuletzt Kontaktsilber aufgetragen, um eine Aufladung im Elektronenstrahl zu verhindern.

Die Analyse der Randschlussqualität erfolgte per REM bei 300-facher Vergrösserung. Dabei wurde der gesamte Rand der zylindrischen Kavität (Ø 4 mm) in 40 Randabschnitte geteilt und für jeden Randabschnitt der prozentuale Anteil an regelmässigem Rand zugeordnet. Im Anschluss wurde die Summe der Prozente durch die Anzahl der Randabschnitte (=40) geteilt. Als regelmässig wurde ein Randabschnitt bewertet, wenn sich dieser ohne erkennbaren Spalt, Unregelmässigkeit oder Randfraktur darstellte. Eine gute Randqualität ist gegeben, wenn der Anteil defektfreien Rands 70% im Median übersteigt. Die Ergebnisse dieser Untersuchung sind für das Konditionierungsmittel B und das Adhäsiv ExciTE F in Tabelle 5 aufgeführt.

**Tabelle 5: Schmelzrandqualität bei einer Klasse I Kavität**

| **Ätzlösung** | **Adhäsiv** | **Anteil perfekter Rand** |
|---|---|---|
| **Email Preparator Blau**¹ | Excite F | 80 - 90 |
| **B** | | 80 - 90 |

| | | |
|---|---|---|
| ¹ Email Preparator Blau, Fa. Ivoclar Vivadent: 37%ige Phosphorsäure (Vergleichsbeispiel) | | |

Wie Tabelle 5 zeigt, ergibt das Konditionierungsmittel B eine vergleichbare Randqualität wie das konventionelle Vorgehen unter Einsatz von 37%iger Phosphorsäure.

### Beispiel 5

### Inhibierung des Kollagenabbaus

Zur Untersuchung des Einflusses von Zirkoniumsalzlösungen auf den durch Metall-Matrix-Proteasen (MMP) induzierten Kollagenabbau wurden bovine Zahnscheiben mit 1mm Dicke mit einer Präzisionskreissäge hergestellt. Die Zahnscheiben wurden durch Einlegen in 2M HCl bei Raumtemperatur vollständig demineralisiert und anschließend mit Citratpuffer säurefrei gewaschen.

Zur Untersuchung der Ätzgel-Wirkung auf die enzymatische Degradation von Kollagen wurde als MMP-Vertreter die Kollagenase Typ I verwendet, wie sie im "Collagenase Substrate Kit" (Fa. Fluka; Nr. 27672/27670) eingesetzt wird.

Referenzproben wurden nach diesem Waschschritt nicht weiter behandelt, sondern direkt untersucht. Testproben hingegen wurden für 60s in 1%ige oder 2%ige Zirkonylchlorid-Lösung getaucht und danach mit destilliertem Wasser sorgfältig abgespült. Die Blindprobe wurde weder mit Kollagenase noch mit der Zirkoniumsalzlösung behandelt.

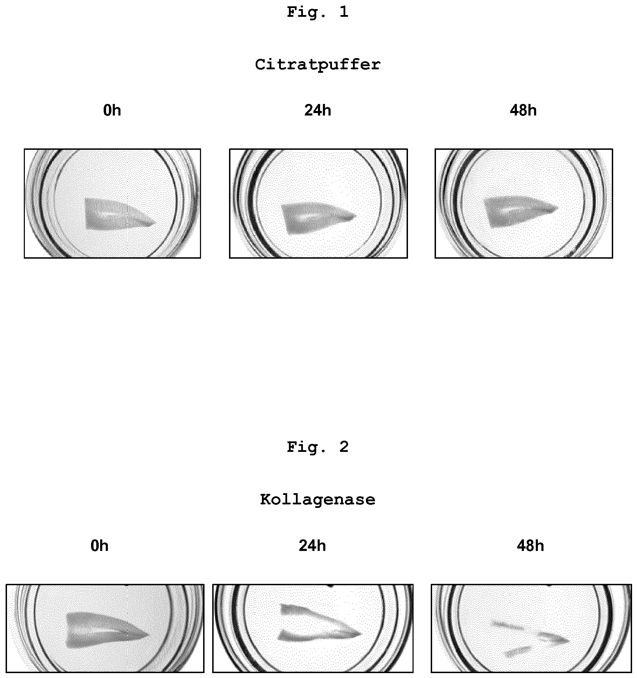

Die Kollagenproben wurden in Petrischalen gegeben und mit einer gepufferten Lösung der Typ I-Kollagenase überschichtet. Inkubation der Proben bei 37°C unter sanfter Agitation durch einen Laborschüttler führte bei den nicht mit der Zirkoniumsalz-Lösung behandelten Proben zu enzymatischen Abbau der demineralisierten Zahnscheiben. Die Figuren 1 bis 4 zeigen die bei diesem Test ermittelten Ergebnisse.

Figur 1 zeigt die Blindprobe ohne Kollagenase und ohne Zirkoniumsalz. Auch nach 48stündiger Lagerung ist keine Änderung der demineralisierten Zahnscheibe erkennbar, d.h. das Kollagen wurde nicht abgebaut.

Figur 2 zeigt, dass die Zugabe von Kollagenase innerhalb von 48h zu einem fast vollständigen Abbau der demineralisierten Zahnscheibe führt. Schon nach 24h ist ein deutlicher Abbau erkennbar.

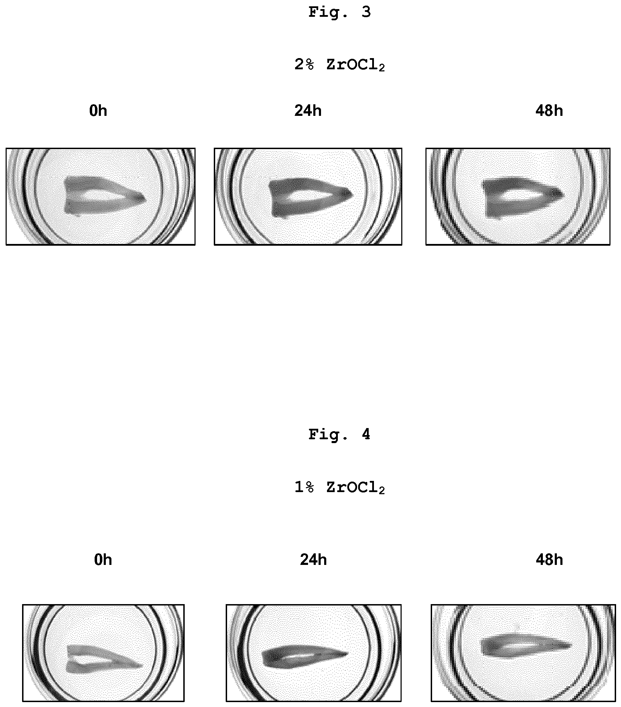

In den Figuren 3 und 4 ist zu erkennen, dass bereits kurzer Kontakt von dentinalem Kollagen mit einer Zirkoniumsalzlösung zu einer Stabilisierung gegen enzymatischen Abbau führt. Auch nach 48h war bei Zirkon-behandelten Proben keine Degradation ersichtlich.

### Beispiel 6

### Bestimmung der Ätzmuster auf Schmelz und Dentin

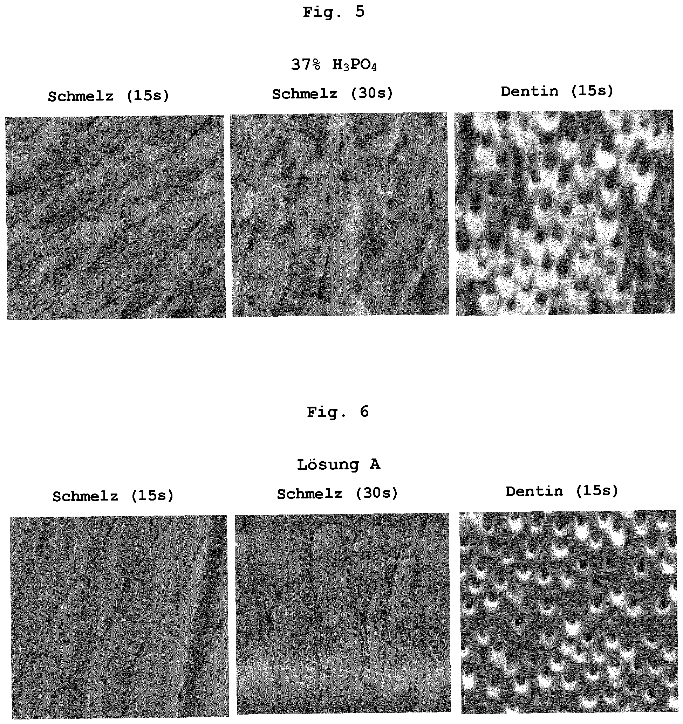

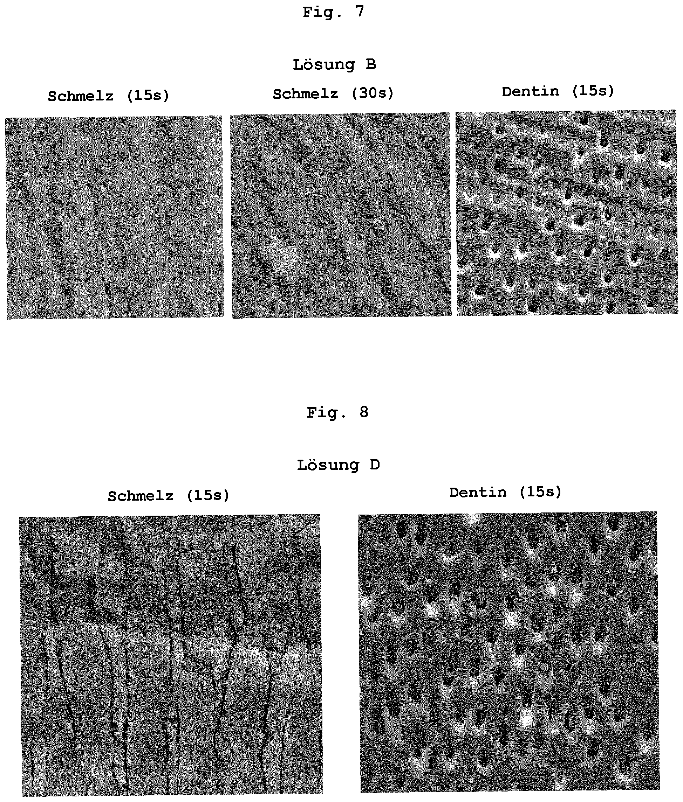

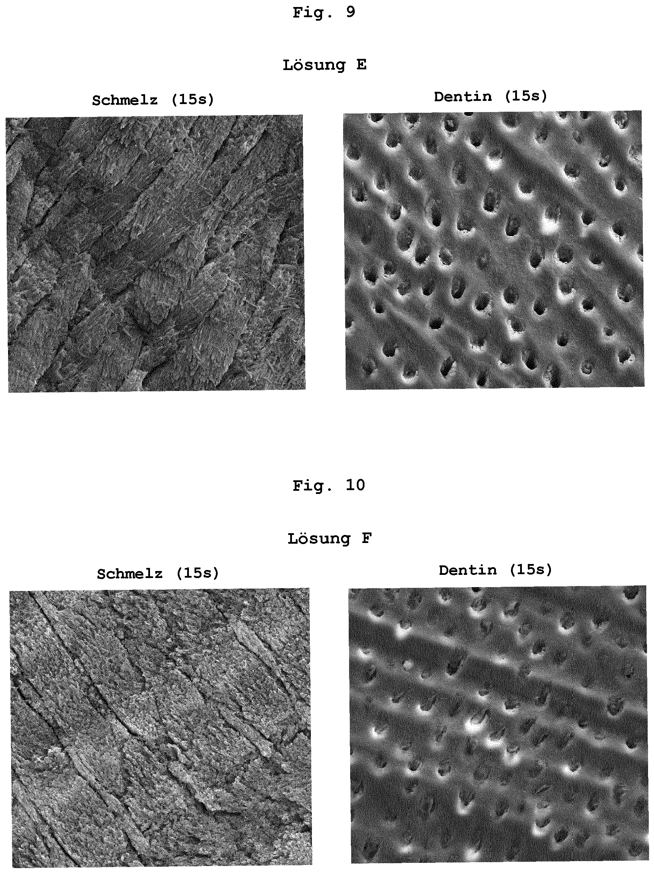

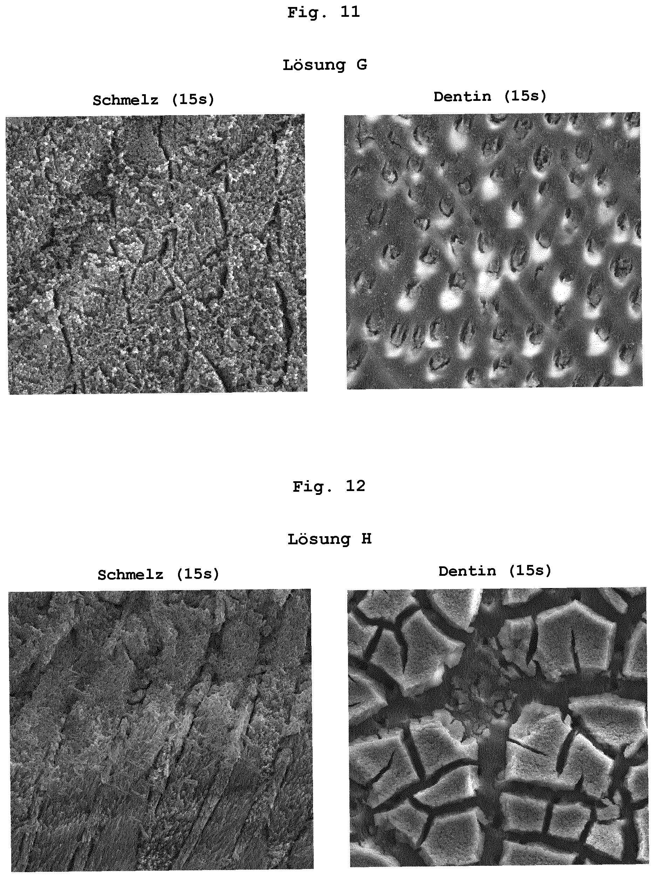

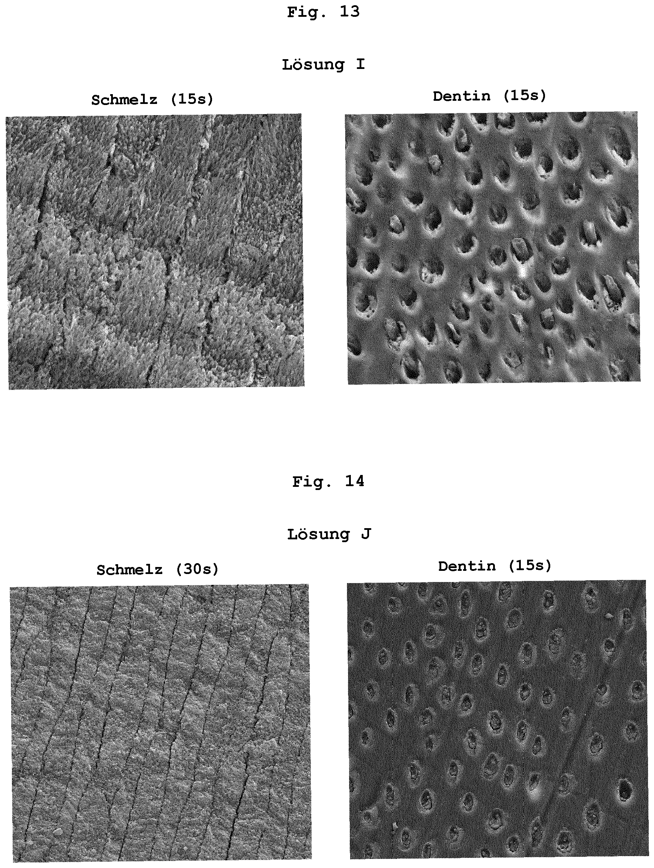

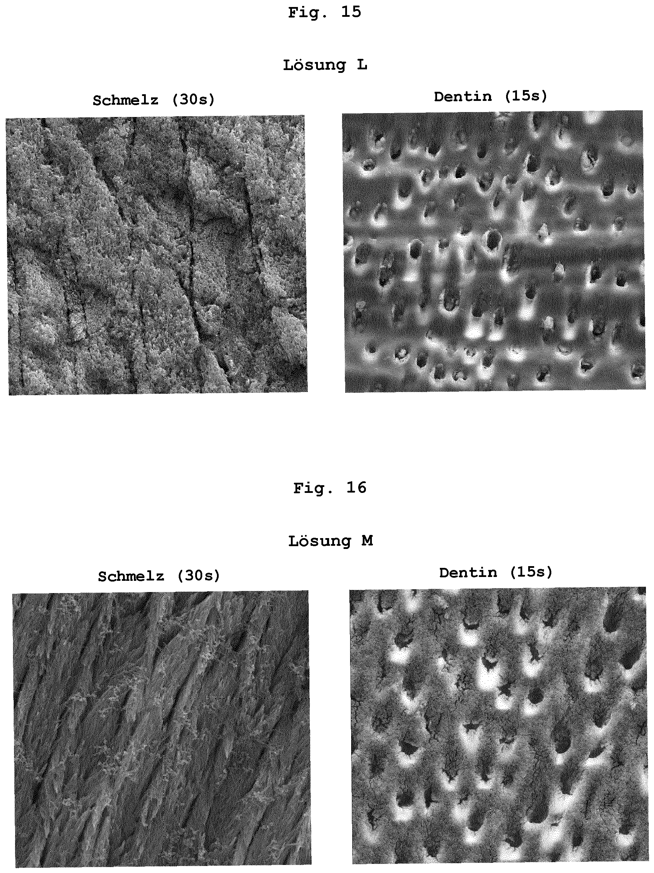

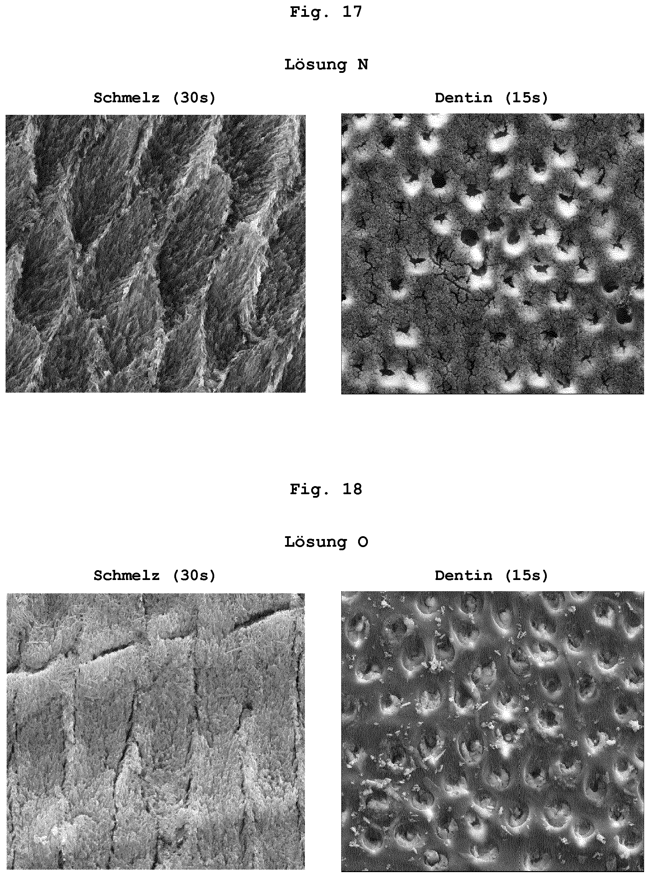

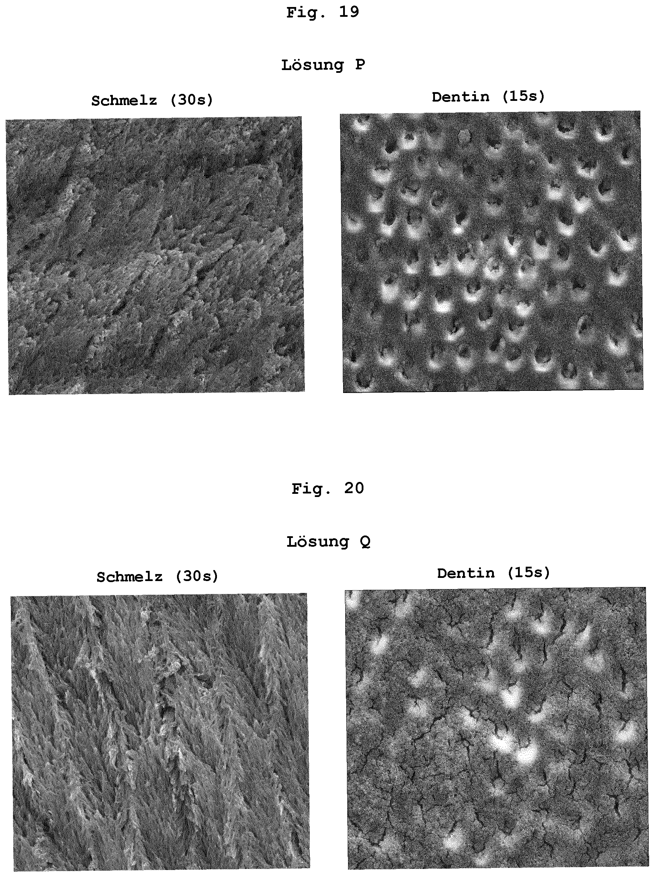

Um die Ausbildung eines mikroretentiven Ätzmusters durch die erfindungsgemässe Konditionierungslösung nachzuweisen, wurden Rinderschmelz und Rinderdentin mit den Formulierungen aus Beispiel 1 kontaktiert, nach der vorgesehenen Einwirkdauer mit Wasser abgespült und die Oberfläche im Rasterelektronenmikroskop untersucht. Als Vergleich diente die konventionell eingesetzte Phosphorsäure. Die Einwirkdauer betrug unabhängig vom Ätzmittel auf Dentin 15s und auf Schmelz 15 bis 30s. Die Ergebnisse bei identischer Vergrösserung sind in den Figuren 5 bis 20 dargestellt.

Die Figuren 5 bis 20 zeigen für Dentin einen deutlichen Unterschied zwischen Phosphorsäure und den erfindungsgemässen Konditionierungsmitteln. So bewirkt Phosphorsäure bereits nach 15s ein Öffnen aller Tubuli durch Entfernen der Schmierschicht und zu deutlichen Demineralisationserscheinungen, erkennbar an weißen Flächen, die sich in Folge der elektrostatischen Aufladung der durch Phosphorsäure dünn geätzten und damit dünn auslaufenden Tubuliränder im Elektronenstrahl bilden (Fig. 5).

Bei Anwendung der erfindungsgemäßen Konditionierungsmittel (Fig. 6-20) hingegen bleiben die Tubuli kurz unterhalb der Dentinoberfläche weitgehend verschlossen. Zu elektrostatischen Aufladungen führende Demineralisationserscheinungen überätzter Tubuliränder sind, anders als nach Phosphorsäurebehandlung, kaum sichtbar. Ein Überätzen des Dentins durch die erfindungsgemäßen Formulierungen kann daher ausgeschlossen werden.

Im Gegensatz zu Dentin zeigen die Fig. 5-20 für Schmelz keinen signifikanten Unterschied zwischen Phosphorsäure und den erfindungsgemässen Konditionierungsmitteln. Alle Metallsalzlösungen führten zur Ausbildung eines retentiven Ätzmusters auf dem Schmelz, welches in allen untersuchten Fällen eine gute Verbundwirkung mit dem aufgebrachten Adhäsiv ermöglicht.

## Patentansprüche

1. Wasserhaltige Lösung, die mindestens ein Übergangs- und/oder Hauptgruppenmetallsalz enthält, zur therapeutischen Verwendung als Konditionierungsmittel für Zähne, wobei das Übergangs- und/oder Hauptgruppenmetallsalz ein Salz von Zr⁴⁺, Hf⁴⁺, Ti⁴⁺ oder Bi³⁺ ist.

2. Lösung nach Anspruch 1, in der das Übergangs- oder Hauptgruppenmetallsalz als Anion Cl⁻, Br⁻, I⁻, NO₃⁻, SO₄²⁻, ggf. als gemischte Salze mit Oxoanionen enthält.

3. Lösung nach einem der Ansprüche 1 bis 2, die als Übergangs- und/oder Hauptgruppenmetallsalz ZrOCl₂, ZrO(NO₃)₂, Zr(SO₄)₂, Hf(SO₄)₂, HfCl₄, Hf(NO₃)₄, TiOSO₄, Bi(NO₃)₃ oder eine Mischung dieser Salze enthält.

4. Lösung nach einem der Ansprüche 1 bis 3, die zusätzlich ein weiteres Metallsalz enthält, das aus den Salzen des Cu²⁺, Zn²⁺, Al³⁺ und Mischungen davon ausgewählt ist.

5. Lösung nach Anspruch 4, die als zusätzliches Metallsalz Al(NO₃)₃, CuSO₄, ZnSO₄ oder eine Mischung davon enthält.

6. Lösung nach einem der Ansprüche 1 bis 5, die keine zugesetzte Säure enthält.

7. Lösung nach einem der Ansprüche 1 bis 6, die 0,5 bis 25 Gew.-% Übergangs- und/oder Hauptgruppenmetallsalz(e) enthält.

8. Lösung nach einem der Ansprüche 1 bis 7, die 2 bis 15 Gew.-% Übergangs- und/oder Hauptgruppenmetallsalz, 36 bis 98 Gew.-% Wasser und 0 bis 49 Gew.-% organisches Lösungsmittel enthält.

9. Lösung nach einem der Ansprüche 1 bis 7, die 4 bis 10 Gew.-% Übergangs- und/oder Hauptgruppenmetallsalz, 42 bis 96 Gew.-% Wasser und 0 bis 48 Gew.-% organisches Lösungsmittel enthält.

10. Lösung nach einem der Ansprüche 1 bis 9, die als organisches Lösungsmittel Glycerin, Ethanol, Ethylenglykol, Propylenglykol, Propandiol, Butandiol und Polyethylenglykol oder eine Mischung davon enthält.

11. Lösung nach einem der Ansprüche 1 bis 10 zur Verwendung als Konditionierungsmittel für die Zahnoberfläche vor der Anwendung dentaler Zemente, Füllungskomposite, Beschichtungsmaterialien oder Verblendmaterialien, bei der Konditionierung der Zahnoberfläche bei der dentalen Restaurationstherapie, bei der Restauration geschädigter Zähne, oder bei der restaurativen Füllungstherapie.

12. Lösung nach einem der Ansprüche 1 bis 10 zur Verwendung als Konditionierungsmittel bei der Befestigung festsitzender Prothetik durch adhäsive Zementierung, bei präventiven Maßnahmen, wie der Versiegelung von Zahnfissuren, oder bei Kieferorthopädische Maßnahmen, wie der Befestigung von Brackets.

13. Verwendung einer Metallsalzlösung gemäß einem der Ansprüche 1 bis 10 zur extraoralen Vorbehandlung der Oberfläche von Dentalrestaurationen, Prothesen, künstlichen Zähnen, Inlays, Onlays, Kronen und Brücken.

## Claims

1. Aqueous solution which comprises at least one transition and/or main group metal salt for therapeutic use as a conditioning agent for teeth, wherein the transition and/or main group metal salt is a salt of Zr⁴⁺, Hf⁴⁺, Ti⁴⁺ or Bi³⁺.

2. Solution according to claim 1 in which the transition and/or main group metal salt comprises Cl⁻, Br⁻, I⁻, NO₃⁻, SO₄²⁻ as the anion, optionally as mixed salts with oxoanions.

3. Solution according to one of claims 1 to 2 which comprises ZrOCl₂, ZrO(NO₃)₂, Zr(SO₄)₂, Hf(SO₄)₂, HfCl₄, Hf(NO₃)₄, TiOSO₄, Bi(NO₃)₃ as the transition and/or main group metal salt or a mixture of these salts.

4. Solution according to one of claims 1 to 3 which further comprises an additional metal salt that is selected from the salts of Cu²⁺, Zn²⁺, Al³⁺ and mixtures thereof.

5. Solution according to claim 4 which comprises Al(NO₃)₃, CuSO₄, ZnSO₄ or a mixture thereof as the additional salt.

6. Solution according to one of claims 1 to 5 which comprises no added acid.

7. Solution according to one of claims 1 to 6 which comprises 0.5 to 25 wt % transition and/or main group metal salt(s).

8. Solution according to one of claims 1 to 7 which comprises 2 to 15 wt % transition and/or main group metal salt, 36 to 98 wt % water and 0 to 49 wt % organic solvent.

9. Solution according to one of claims 1 to 7 which comprises 4 to 10 wt % transition and/or main group metal salt, 42 to 96 wt % water and 0 to 48 wt % organic solvent.

10. Solution according to one of claims 1 to 9 which comprises glycerol, ethanol, ethylene glycol, propylene glycol, propane diol, butane diol and polyethylene glycol or a mixture thereof as the organic solvent.

11. Solution according to one of claims 1 to 10 for use as a conditioning agent for the tooth surface before the application of dental cements, filling composites, coating materials or veneering materials, in the conditioning of the tooth surface in dental restoration therapy, in the restoration of damaged teeth, or in restorative filling therapy.

12. Solution according to one of claims 1 to 10 for use as a conditioning agent in the securing of fixed prosthodontics by adhesive cementing, in preventive measures such as the sealing of tooth fissures, or in orthodontic measures such as the securing of brackets.

13. Use of a metal salt solution according to one of claims 1 to 10 for the extraoral pre-treatment of the surface of dental restorations, prostheses, artificial teeth, inlays, onlays, crowns and bridges.

## Revendications

1. Solution aqueuse, qui contient au moins un sel de métal de transition et/ou de métal de groupes principaux, destinée à l'utilisation thérapeutique en tant qu'agent de conditionnement pour dents, le sel de métal de transition et/ou de métal de groupes principaux étant un sel de Zr⁴⁺, Hf⁴⁺ , Ti⁴⁺ ou Bi³⁺.

2. Solution selon la revendication 1, dans laquelle le sel de métal de transition et/ou de métal de groupes principaux contient en tant qu'anion Cl⁻, Br⁻, I⁻, NO₃⁻, SO₄²⁻, éventuellement sous forme de sels mixtes avec des oxoanions.

3. Solution selon l'une quelconque des revendications 1 et 2, qui contient en tant que sel de métal de transition et/ou de métal de groupes principaux du ZrOCl₂, ZrO(NO₃)₂, Zr(SO₄)₂, Hf(SO₄)₂, HfCl₄, Hf(NO₃)₄, TiOSO₄, Bi(NO₃)₃ ou un mélange de ces sels.

4. Solution selon l'une quelconque des revendications 1 à 3, qui contient en plus un autre sel métallique qui est choisi parmi les sels du Cu²⁺, Zn²⁺, Al³⁺ et des mélanges de tels sels.

5. Solution selon la revendication 4, qui contient en tant que sel métallique supplémentaire Al(NO₃)₃, CuSO₄, ZnSO₄ ou un mélange de tels sels.

6. Solution selon l'une quelconque des revendications 1 à 5, qui ne contient aucun acide ajouté.

7. Solution selon l'une quelconque des revendications 1 à 6, qui contient 0,5 à 25 % en poids de sel(s) de métal/métaux de transition et/ou de métal/métaux de groupes principaux.

8. Solution selon l'une quelconque des revendications 1 à 7, qui contient 2 à 15 % en poids de sel de métal de transition et/ou de métal de groupes principaux, 36 à 98 % en poids d'eau et 0 à 49 % en poids de solvant organique.

9. Solution selon l'une quelconque des revendications 1 à 7, qui contient 4 à 10 % en poids de sel de métal de transition et/ou de métal de groupes principaux, 42 à 96 % en poids d'eau et 0 à 48 % en poids de solvant organique.

10. Solution selon l'une quelconque des revendications 1 à 9, qui contient en tant que solvant organique du glycérol, de l'éthanol, de l'éthylèneglycol, du propylèneglycol, du propanediol, du butanediol et du polyéthylèneglycol ou un mélange de ceux-ci.

11. Solution selon l'une quelconque des revendications 1 à 10, destinée à l'utilisation en tant que conditionneur pour la surface dentaire avant l'application de ciments, composites d'obturation, matériaux de revêtement ou matériaux de placage dentaires, dans le conditionnement de la surface dentaire dans le traitement de restauration dentaire, dans la restauration de dents abîmées, ou dans le traitement d'obturation restauratrice.

12. Solution selon l'une quelconque des revendications 1 à 10, pour utilisation en tant que conditionneur dans la fixation de prothèse fixe par cimentation adhésive, dans des mesures préventives, telles que le scellement de fissures dentaires, ou dans des travaux d'orthodontie, tels que la fixation de brackets.

13. Utilisation d'une solution de sel métallique selon l'une quelconque des revendications 1 à 10, pour le prétraitement extra-oral de la surface de restaurations dentaires, prothèses, dents artificielles, inlays, onlays, couronnes et bridges.
